# EUROPEAN PATENT SPECIFICATION

(11) **EP 3 370 600 B1**
(45) Date of publication and mention of the grant of the patent: **03.04.2024**
(21) Application number: 16795443.7
(22) Date of filing: 31.10.2016
(51) Int. Cl.: A61B 5/00, A61B 5/024, A61B 5/029, A61B 5/021, A61B 5/083, A61B 5/145

(54) **VITAL INFORMATION DISPLAYING APPARATUS**
VITALINFORMATIONSANZEIGEVORRICHTUNG
APPAREIL D'AFFICHAGE D'INFORMATIONS VITALES

(30) Priority: 02.11.2015 JP 2015215742
(43) Date of publication of application: 12.09.2018
(73) Proprietor: NIHON KOHDEN CORPORATION, Shinjuku-ku, Tokyo 1618560 (JP)
(72) Inventor: TANAKA, Rie, Tokyo 161-8560 (JP)
(74) Representative: Grünecker Patent- und Rechtsanwälte PartG mbB
(86) International application number: PCT/JP2016/004772
(87) International publication number: WO 2017/077702

(56) References cited:
- EP-A1- 2 314 211
- EP-A2- 2 685 398
- WO-A1-2009/094700
- JP-B1- 3 948 677
- US-A1- 2006 025 698
- US-A1- 2010 099 964

## Description

### Technical Field

The present disclosure relates to an apparatus for displaying vital information.

### Background Art

For example, PTL1 discloses an apparatus for displaying vital information, wherein a coordinate plane formed by a first coordinate axis which extends in a first direction, and a second coordinate axis which extends in a second direction perpendicular to the first direction is displayed on a displaying section. A first vital parameter is associated with the first coordinate axis, and a second vital parameter is associated with the second coordinate axis.

Namely, a combination of measured values of the first and second vital parameters at a certain time point is displayed as a point on the coordinate plane. Temporal changes of the measured values of the vital parameters are indicated as a movement of the point on the coordinate plane. Other apparatuses for displaying temporal changes of first and second vital parameters are disclosed in PTL2 and PTL3.

### Citation List

### Patent Literature

PTL 1: Japanese Patent Publication No. 2009-219829A
PTL2: JP3948677 B1; PTL3: EP2685398 A2 **Summary of Invention**

### Technical Problem

According to an aspect of the present disclosure, an apparatus for displaying vital information can configured to improve the visibility of temporal changes of measured values of a plurality of vital parameters that are indicated as a movement of a point on a coordinate plane.

### Solution to Problem

According to a first aspect of the present disclosure, a vital information displaying apparatus can comprise:
a displaying section;
a display controlling section configured to cause the displaying section to display a coordinate plane formed by a first coordinate axis extending in a first direction and a second coordinate axis extending in a second direction perpendicular to the first direction; and
a parameter setting section configured to selectively determine a first vital parameter to be associated with the first coordinate axis and a second vital parameter to be associated with the second coordinate axis,
wherein the display controlling section is configured to cause items to be displayed on the coordinate plane in the displaying section, the items including:
   a first reference line indicating a first reference value of the first vital parameter and extending in the second direction;
   a second reference line indicating a second reference value of the fist vital parameter and extending in the second direction;
   a third reference line indicating a first reference value of the second vital parameter and extending in the first direction;
   a fourth reference line indicating a second reference value of the second vital parameter extending in the first direction;
   a first symbol indicating a combination of a value of the first vital parameter and a value of the second vital parameter that are measured at a first time point; and
   a plurality of second symbols each indicating a combination of a value of the first vital parameter and a value of the second vital parameter that are measured at one of second time points that are before the first time point;
   wherein the display controlling section is configured to cause a region defined inside the first reference line, the second reference line, the third reference line and the fourth reference line to be displayed on such a position that includes a center of the coordinate plane.

The region defined inside the first, second, third and fourth reference lines may represent a range where the values of both the selected first and second vital parameters are deemed to be normal. The user can monitor the condition of the subject from the viewpoint of whether the first symbol is within the region or not.

In the above vital information displaying apparatus, the first and second vital parameters which are to be displayed are not determined in advance. Accordingly, the first, second, third and fourth reference lines are not to be fixed. With this configuration, however, the region is displayed in the portion including the center of the coordinate plane 20 irrespective of the kinds of the selected first and second vital parameters. Consequently, the user can always see the targeted region in the vicinity of the center of the coordinate plane.

Therefore, the visibility of information with higher importance can be improved while ensuring the degree of freedom of vital information displayed on the displaying section. Namely, it is possible to improve the visibility of the vital information displaying apparatus on which temporal changes of measured values of a plurality of vital parameters are indicated as a movement of a point on the coordinate plane.

### Brief Description of Drawings

[fig.1] Fig. 1 illustrates the functional configuration of a vital information displaying apparatus according to one embodiment.
[fig.2]Fig. 2 illustrates one display mode of a displaying section of the vital information displaying apparatus.
[fig.3]Fig. 3 illustrates another display mode of the displaying section of the vital information displaying apparatus.
[fig.4]Fig. 4 illustrates another display mode of the displaying section of the vital information displaying apparatus.
[fig.5]Fig. 5 illustrates another display mode of the displaying section of the vital information displaying apparatus.
[fig.6A] Fig. 6A illustrates one example of symbols indicating a change amount displayed in the displaying section.
[fig.6B]Fig. 6B illustrates another example of the symbols indicating the change amount displayed in the displaying section.
[fig.7A]Fig. 7A illustrates another example of the symbols indicating the change amount displayed in the displaying section.
[fig.7B]Fig. 7B illustrates another example of the symbols indicating the change amount displayed in the displaying section.
[fig.8A]Fig. 8A illustrates illustrating another example of the symbols indicating the change amount displayed in the displaying section.
[fig.8B]Fig. 8B illustrates another example of the symbols indicating the change amount displayed in the displaying section.
[fig.8C]Fig. 8C illustrates another example of the symbols indicating the change amount displayed in the displaying section.
[fig.9]Fig. 9 illustrates another display mode of the displaying section of the vital information displaying apparatus.

### Description of Embodiments

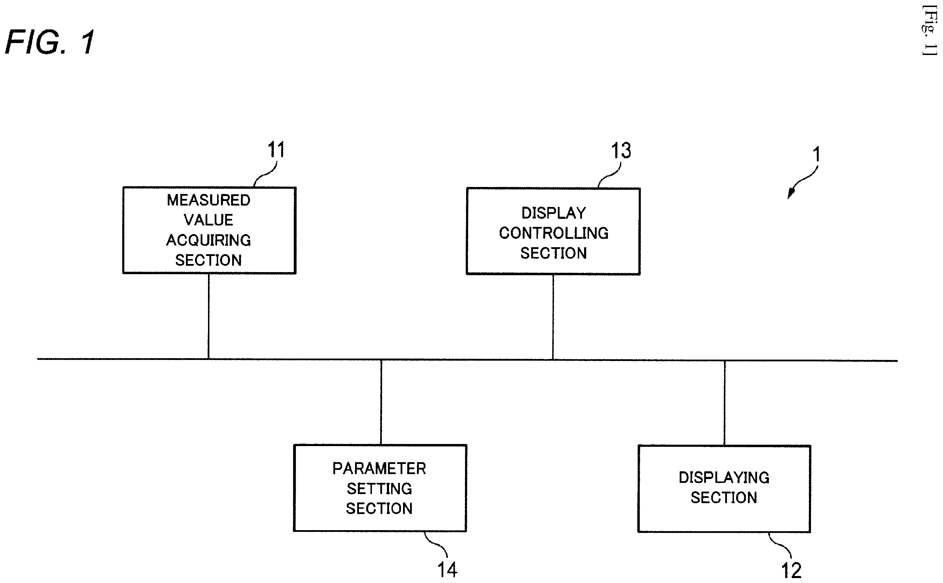

Embodiments will be described in detail with reference to the accompanying drawings. Fig. 1 illustrates the functional configuration of a vital information displaying apparatus 1 according to one embodiment. The vital information displaying apparatus 1 comprises a measured value acquiring section 11, a displaying section 12, a display controlling section 13, and a parameter setting section 14. These components are connected so as to be able to communicate signals and data to one another. The connection may be wired or wireless.

The measured value acquiring section 11 acquires measured values of various vital parameters which are acquired through various sensors attached to a subject. When the sensors are directly connected to the vital information displaying apparatus 1, the outputs of the sensors can be directly input to the measured value acquiring section 11. Alternatively, the outputs of the sensors may be supplied to an external measuring apparatus to be acquired as measured values, and the measured values may be input to the measured value acquiring section 11 through wired or wireless communication. Namely, the vital information displaying apparatus 1 may have any configuration as far as measured values of vital parameters which are acquired through the measured value acquiring section 11 can be displayed on the displaying section 12.

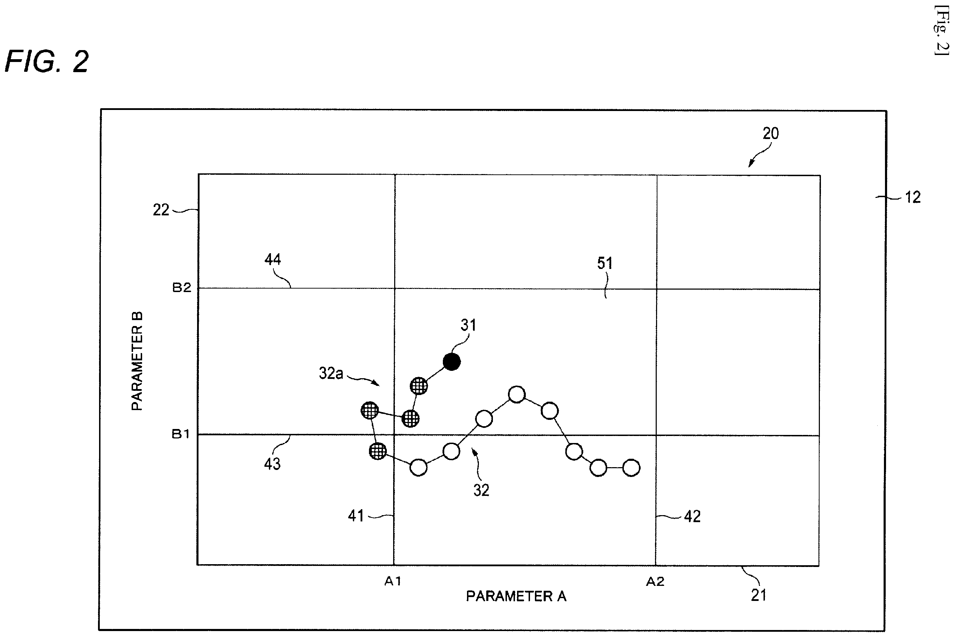

The display controlling section 13 causes a coordinate plane 20 to be displayed on the displaying section 12. Fig. 2 illustrates an example of the coordinate plane 20 displayed on the displaying section 12. The coordinate plane 20 is formed by a first coordinate axis 21 and a second coordinate axis 22. The first coordinate axis 21 extends in the horizontal direction (an example of the first direction), and the second coordinate axis 22 extends in the vertical direction (an example of the second direction). The vertical direction is perpendicular to the horizontal direction.

Both the first coordinate axis 21 and the second coordinate axis 22 are not a time axis. One of a plurality of vital parameters (first vital parameter) acquired by the measured value acquiring section 11 is associated with the first coordinate axis 21. Another one of the plurality of vital parameters (second vital parameter) acquired by the measured value acquiring section 11 is associated with the second coordinate axis 22. Examples of the plurality of vital parameters include blood pressure, heart rate, arterial oxygen saturation, carbon dioxide concentration in expiration, cardiac output, positive end-expiratory pressure, mean airway pressure, inspired oxygen concentration, local oxygen saturation, and body temperature.

Examples of a combination of a pair of vital parameters which are respectively associated with the first and second coordinate axes 21, 22 are combinations of blood pressure and heart rate, positive end-expiratory pressure and inspired oxygen concentration, arterial oxygen saturation and inspired oxygen concentration, local oxygen saturation and blood pressure, and cardiac output and blood pressure.

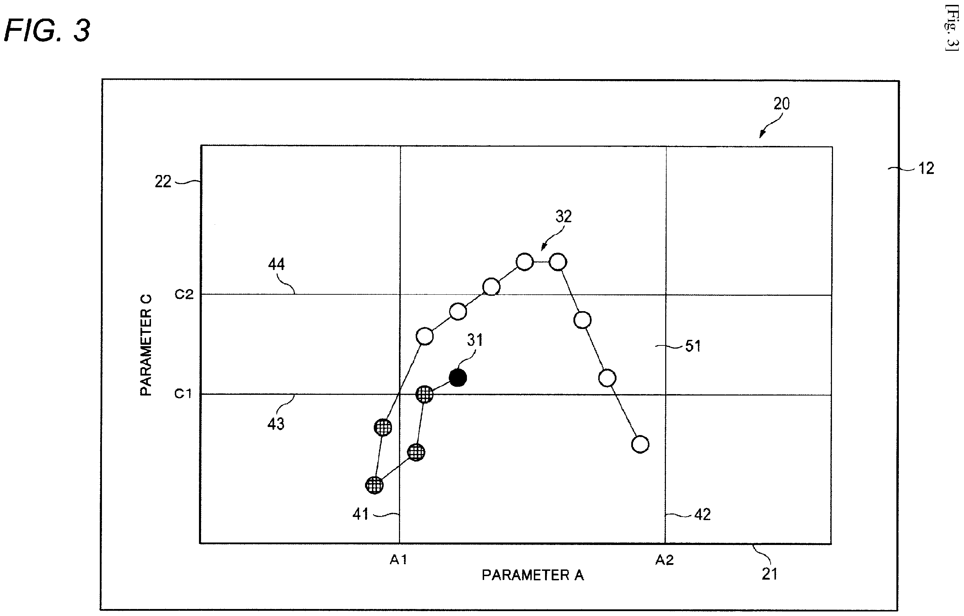

The parameter setting section 14 selectively determines the first vital parameter which is to be associated with the first coordinate axis 21, and the second vital parameter which is to be associated with the second coordinate axis 22. The first and second vital parameters may be changed. At least one of the first and second vital parameters may be automatically determined in accordance with measured values of a plurality of parameters acquired by the measured value acquiring section 11, or may be selected by the user. In the example illustrated in Fig. 2, a parameter A is selected as the first vital parameter, and a parameter B is selected as the second vital parameter. In an example illustrated in Fig. 3, the parameter A is selected as the first vital parameter, and a parameter C is selected as the second vital parameter.

The display controlling section 13 causes a first plot 31 (an example of the first symbol) indicating a combination of measured values of the first and second vital parameters at the present time point (an example of the first time point), to be displayed on the coordinate plane 20 of the displaying section 12.

The display controlling section 13 causes a plurality of second plots 32 (an example of the second symbols) respectively indicating combinations of measured values of the first and second vital parameters at a plurality of past time points (an example of the second time points), to be displayed on the coordinate plane 20 of the displaying section 12. The color of the first plot 31 is different from the colors of the second plots 32.

With this the configuration, without using a time axis in the coordinate plane 20, it is possible to recognize temporal changes of measured values of the vital parameters. The number of the second plots 32 which are to be displayed on the coordinate plane 20 can be adequately determined and changed. In the examples shown in Figs. 2 and 3, the number of the second plots 32 is 12. The interval between the present time point at which the first plot 31 is displayed and the past time point at which the last second plot 32 was displayed is equal to the intervals between the past time points which are respectively associated with the second plots 32. The intervals can be adequately determined and changed. In the examples illustrated in Figs. 2 and 3, the intervals are equal to 5 minutes. In the illustrated example, namely, changes of measured values of the vital parameters which are at 5-minute intervals during the past one hour are displayed.

As illustrated in Fig. 2, the display controlling section 13 causes a first reference line 41, a second reference line 42, a third reference line 43, and a fourth reference line 44 to be displayed on the coordinate plane 20. The first reference line 41 indicates a first reference value A1 of the parameter A, and extends in parallel to the second coordinate axis 22. The second reference line 42 indicates a second reference value A2 of the parameter A, and extends in parallel to the second coordinate axis. The third reference line 43 indicates a first reference value B1 of the parameter B, and extends in parallel to the first coordinate axis 21. The fourth reference line 44 indicates a second reference value B2 of the parameter B, and extends in parallel to the first coordinate axis 21.

The first reference value A1 of the parameter A is the lower limit of a range in which the value of the parameter A is deemed to be normal. The second reference value A2 of the parameter A is the upper limit of the range in which the value of the parameter A is deemed to be normal.

The first and second reference values A1, A2 of the parameter A may be stored in advance in a storage section which is not shown. In this case, when the parameter A is selected as the first vital parameter by the parameter setting section 14, the display controlling section 13 reads out the first and second reference values A1, A2 from the storage section, and causes the first and second reference lines 41, 42 respectively corresponding to the read-out first and second reference values A1, A2 to be displayed on the coordinate plane 20 of the displaying section 12.

Alternatively, the first and second reference values A1, A2 may be manually input by the user. Alternatively, the first and second reference values A1, A2 which are read out by the display controlling section 13 may be changed by the user through an appropriate user interface after the first and second reference lines 41, 42 are displayed.

The first reference value B1 of the parameter B is the lower limit of a range in which the value of the parameter B is deemed to be normal. The second reference value B2 of the parameter B is the upper limit of the range in which the value of the parameter B is deemed to be normal.

The first and second reference values B 1, B2 of the parameter B may be stored in advance in the storage section which is not shown. In this case, when the parameter B is selected as the second vital parameter by the parameter setting section 14, the display controlling section 13 reads out the first and second reference values B1, B2 from the storage section, and causes the third and fourth reference lines 43, 44 respectively corresponding to the read-out first and second reference values B1, B2 to be displayed on the coordinate plane 20 of the displaying section 12.

Alternatively, the first and second reference values B1, B2 may be manually input by the user. Alternatively, the first and second reference values B1, B2 which are read out by the display controlling section 13 may be changed by the user through an appropriate user interface after the third and fourth reference lines 43, 44 are displayed.

In the example illustrated in Fig. 3, the third reference line 43 indicates a first reference value C1 of the parameter C, and the fourth reference line 44 indicates a second reference value C2 of the parameter C.

The first reference value C1 of the parameter C is the lower limit of a range in which the value of the parameter C is deemed to be normal. The second reference value C2 of the parameter C is the upper limit of the range in which the value of the parameter C is deemed to be normal.

The first and second reference values C1, C2 of the parameter C may be stored in advance in the storage section which is not shown. In this case, when the parameter C is selected as the second vital parameter by the parameter setting section 14, the display controlling section 13 reads out the first and second reference values C1, C2 from the storage section, and causes the third and fourth reference lines 43, 44 respectively corresponding to the read-out first and second reference values C1, C2, to be displayed on the coordinate plane 20 of the displaying section 12.

Alternatively, the first and second reference values C1, C2 may be manually input by the user. Alternatively, the first and second reference values C1, C2 which are read out by the display controlling section 13 may be changed by the user through an appropriate user interface after the third and fourth reference lines 43, 44 are displayed.

As apparent from Figs. 2 and 3, the display controlling section 13 causes a region 51 which is defined inside the first, second, third, and fourth reference lines 41, 42, 43, 44 on the coordinate plane 20, to be displayed in a portion including the center of the coordinate plane 20.

The region which is defined inside the first and second reference lines 41, 42 represents the range in which the value of the first vital parameter is deemed to be normal. The region which is defined inside the third and fourth reference lines 43, 44 represents the range in which the value of the second vital parameter is deemed to be normal. Therefore, the region 51 represents a range where the values of both the selected first and second vital parameters are deemed to be normal. The user monitors the condition of the subject from the viewpoint of whether the first plot 31 is within the region 51 or not.

In the vital information displaying apparatus 1 of this embodiment, the first and second vital parameters which are to be displayed are not determined in advance. Accordingly, the positions of the first, second, third, and fourth reference lines 41, 42, 43, 44 are not to be fixed. With this configuration, however, the region 51 is displayed in the portion including the center of the coordinate plane 20 irrespective of the kinds of the selected first and second vital parameters. Consequently, the user can always see the targeted region 51 in the vicinity of the center of the coordinate plane 20.

Therefore, the visibility of information with higher importance can be improved while ensuring the degree of freedom of vital information displayed on the displaying section 12. Namely, it is possible to improve the visibility of the vital information displaying apparatus 1 on which temporal changes of measured values of a plurality of vital parameters are indicated as a movement of a point on the coordinate plane 20.

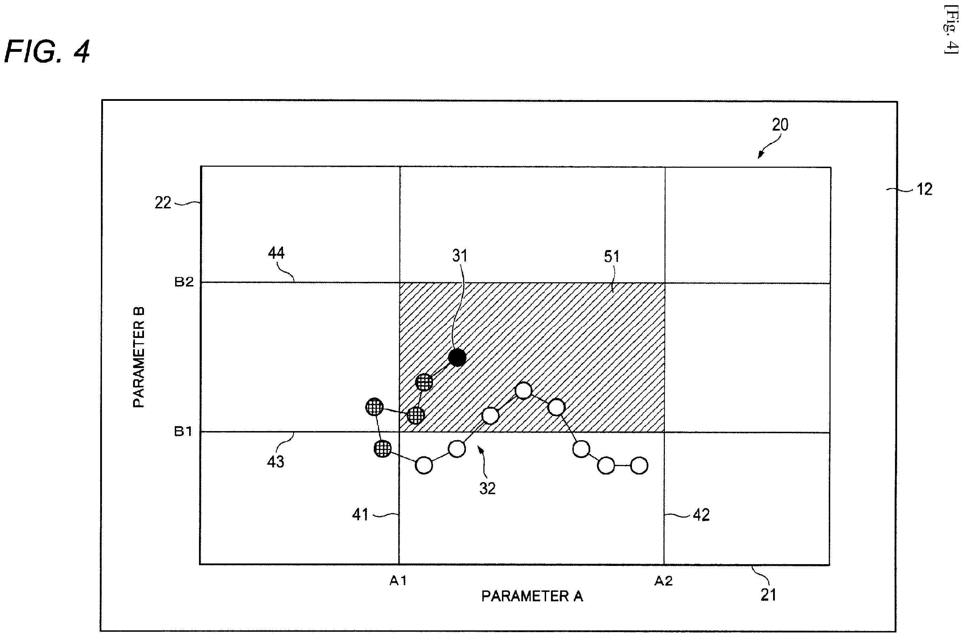

The display controlling section 13 may differentiate the display mode of the region 51 from that of the other portion of the coordinate plane 20. As illustrated in Fig. 4, for example, the display controlling section 13 may differentiate the color of the region 51 from that of the other portion of the coordinate plane 20.

With this configuration, the visibility of information with higher importance can be further improved. Therefore, it is possible to further improve the visibility of the vital information displaying apparatus 1 on which temporal changes of measured values of a plurality of vital parameters are indicated as a movement of a point on the coordinate plane 20.

The coordinate plane 20 is divided into nine regions by the first, second, third, and fourth reference lines 41, 42, 43, 44. The display controlling section 13 may differentiate the display mode of at least one of the nine regions, from the display modes of the other regions of the coordinate plane 20, in accordance with an input from the user. The region(s) where the display mode is to be changed may be designated through an adequate user interface such as a button, a mouse, or a touch panel.

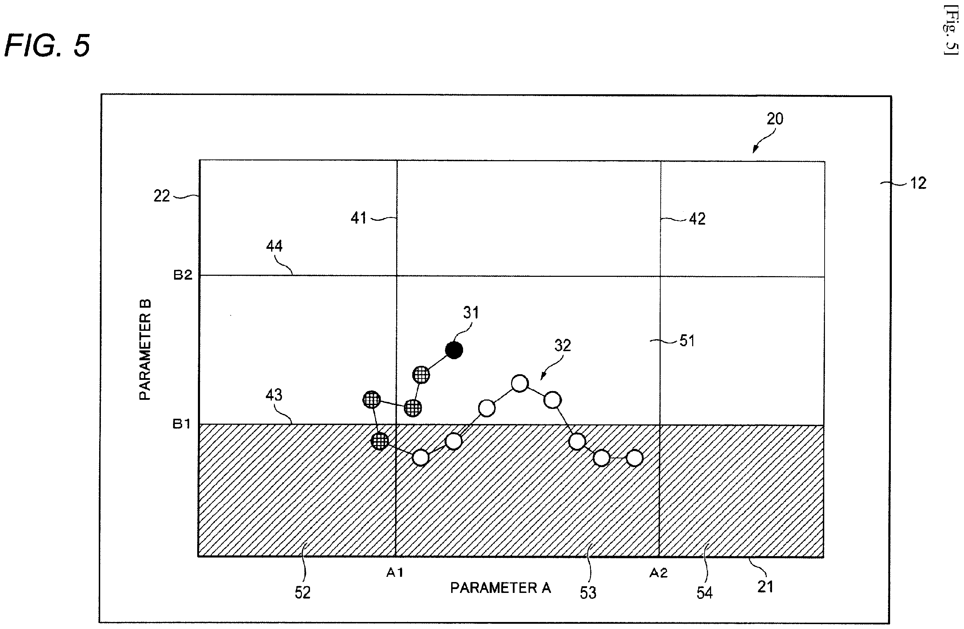

As illustrated in Fig. 5, for example, the display controlling section 13 may differentiate the color of regions 52, 53, 54 which are located below the region 51 from the colors of the other portions in the coordinate plane 20. According to the display mode, for example, follow-up can be easily performed from the viewpoint of whether the parameter B is smaller than the first reference value B1 or not.

With this configuration, therefore, the degree of freedom of the follow-up can be enhanced while ensuring the visibility of information with higher importance. Therefore, it is possible to further improve the visibility of the vital information displaying apparatus 1 on which temporal changes of measured values of a plurality of vital parameters are indicated as a movement of a point on the coordinate plane 20.

The display controlling section 13 may cause the color of at least one of the plurality of second plots 32 to be changed in accordance with the time period elapsing until the present time point. As illustrated in Fig. 2, for example, the display controlling section 13 may cause the colors of four second plots 32a, which are closer to the present time point among the twelve second plots 32, to be changed. The number of second plots 32 which are to be changed, and that of the used colors may be adequately determined. For example, the colors of second plots 32 associated with time points until 30 minutes before the present time point may be changed. Alternatively, second plots 32 associated with time points until 20 minutes before the present time point may be displayed in a first color, second plots 32 associated with time points from 25 to 40 minutes before the present time point may be displayed in a second color, and second plots 32 associated with time points from 45 to 60 minutes before the present time point may be displayed in a third color.

With this configuration, without using a time axis, it is possible to easily recognize temporal changes of measured values of the vital parameters. Therefore, it is possible to further improve the visibility of the vital information displaying apparatus 1 on which temporal changes of measured values of a plurality of vital parameters are indicated as a movement of a point on the coordinate plane 20.

The display controlling section 13 may cause an arrow indicating a first amount of variation of measured values of the first vital parameter (an example of the third symbol) and an arrow indicating a second amount of variation of measured values of the second vital parameter from one of a plurality of past time points to the present time point (an example of the third symbol) to be displayed on the displaying section 12.

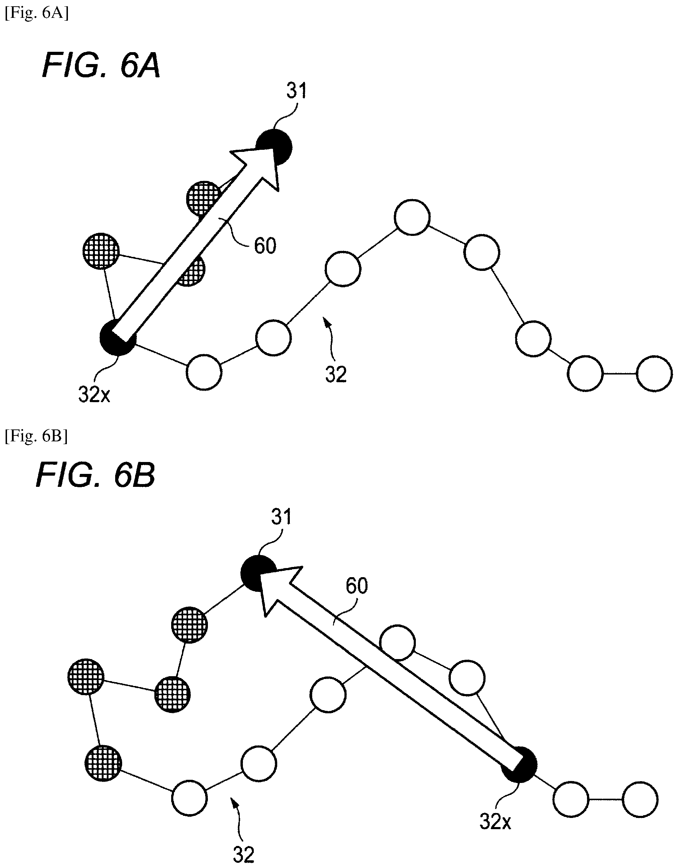

In an example illustrated in Fig. 6A, for example, it is designated the second plot 32x corresponding to the values of the first and second vital parameters measured at the time point that is 20 minutes before the present time point. The first amount of variation of the first vital parameter, and second amount of variation of the second vital parameter during the 20 minutes are indicated by an arrow 60 (an example of the third symbol). The arrow 60 extends from the second plot 32x toward the first plot 31. Each of the first and second amounts of changes includes information of the absolute value and increase or decrease of the amount of variation. Therefore, the first amount of variation can be expressed by a vector which extends in parallel to the first coordinate axis 21, and the second amount of variation can be expressed by a vector which extends in parallel to the second coordinate axis 22. The arrow 60 corresponds to the resultant vector of the vector indicating the first amount of variation and the vector indicating the second amount of variation.

In an example illustrated in Fig. 6B, it is designated the second plot 32x corresponding to the values of the first and second vital parameters measured at the time point that is 50 minutes before the present time point. The past time point which is the initial point may be designated through an adequate user interface such as a button, a mouse, or a touch panel.

With this configuration, even when the second plots 32 are displayed in a large number, the condition change of the subject from an arbitrary past time point to the present time point can be intuitively visible. Therefore, it is possible to further improve the visibility of the vital information displaying apparatus 1 on which the temporal change of the measured values of a plurality of vital parameters are indicated as a movement of the point on the coordinate plane 20.

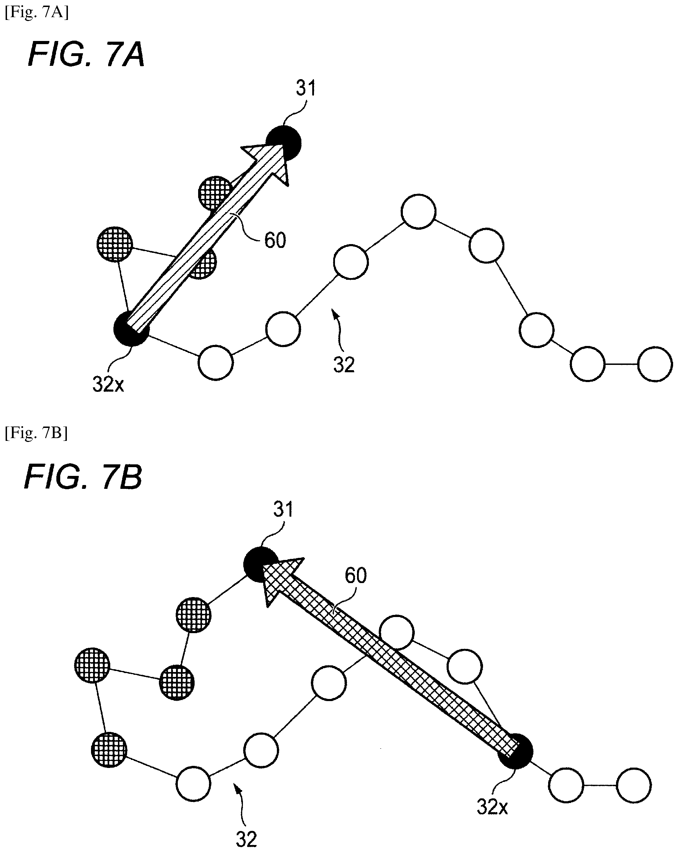

In this case, the display controlling section 13 may cause the arrow 60 to be displayed in a color corresponding to the larger one of the first and second amounts of changes. In an example illustrated in Fig. 7A, for example, the second amount of variation is larger than the first amount of variation. The arrow 60 in the figure is displayed in a color which is determined in advance so as to correspond to the second vital parameter. On the other hand, in an example illustrated in Fig. 7B, the first amount of variation is larger than the second amount of variation. The arrow 60 in the figure is displayed in a color which is determined in advance so as to correspond to the first vital parameter.

With this configuration, a vital parameter in which the amount of variation is larger, and which usually has higher importance can be specified while ensuring intuitive visibility. Therefore, more detailed information can be provided to the user while improving the visibility of the vital information displaying apparatus 1 on which temporal changes of measured values of a plurality of vital parameters are indicated as a movement of a point on the coordinate plane 20.

Alternatively, the display controlling section 13 may be configured so as to cause a first arrow indicating the first amount of variation of measured values of the first vital parameter (an example of the third symbol) and a second arrow indicating the second amount of variation of measured values of the second vital parameter from one of a plurality of past time points to the present time point (an example of the third symbol) to be displayed on the displaying section 12.

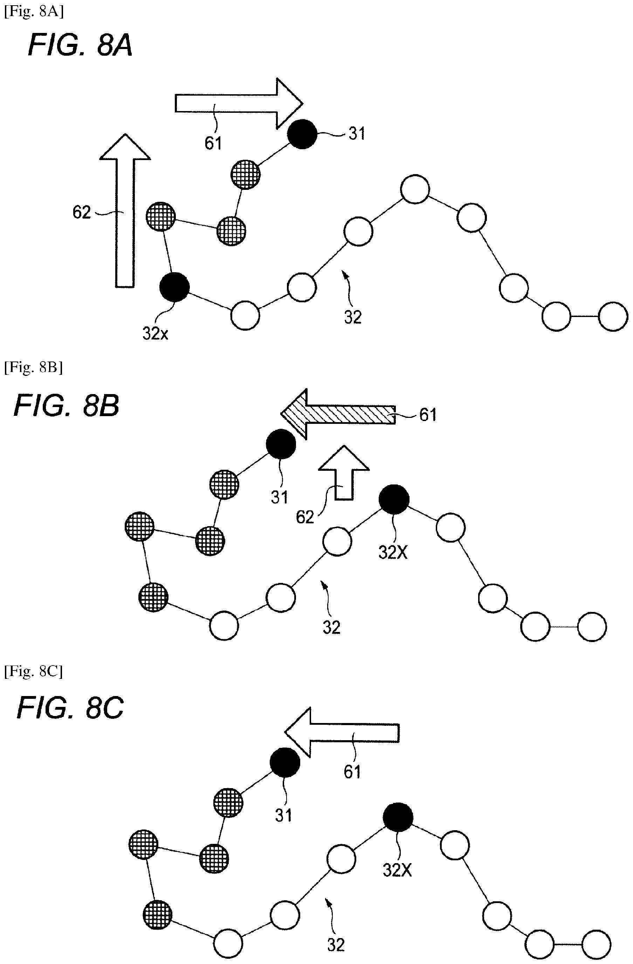

In an example illustrated in Fig. 8A, for example, it is designated the second plot 32x corresponding to the values of the first and second vital parameters measured at the time point that is 20 minutes before the present time point. The first amount of variation of the first vital parameter during the 20 minutes is indicated by a first arrow 61. The first arrow 61 extends from the second plot 32x toward the first plot 31 in parallel to the first coordinate axis 21. The second amount of variation of the second vital parameter during the 20 minutes is indicated by a second arrow 62. The second arrow 62 extends from the second plot 32x toward the first plot 31 in parallel to the second coordinate axis 22.

With this configuration, even when the second plots 32 are displayed in a large number, information relating to the condition change of the subject from an arbitrary past time to the present time point is visible in more detail and intuitively. Therefore, more detailed information can be provided to the user while improving the visibility of the vital information displaying apparatus 1 on which temporal changes of measured values of a plurality of vital parameters are indicated as a movement of a point on the coordinate plane 20.

In this case, the display controlling section 13 may cause the color of the first arrow 61 to be changed in accordance with the values of the first amount of variation, and the color of the second arrow 62 to be changed in accordance with the value of the second amount of variation.

In an example illustrated in Fig. 8B, for example, it is designated the second plot 32x corresponding to the values of the first and second vital parameters measured at the time point that is 40 minutes before the present time point. In the example, the first amount of variation is larger than a predetermined value, and the color of the first arrow 61 is changed. The kinds and number of the colors may be adequately determined. Preferably, colors which easily attract user attention, such as red and yellow may be used. When the second amount of variation is larger than a predetermined value, the color of the second arrow 62 may be similarly changed.

Usually, the fact that the amount of variation of measurement vales of a vital parameter is large represents information with higher importance. According to the above-described configuration, the first arrow 61 and the second arrow 62 are displayed, and hence information with higher importance is easily provided to the user even when the amount of information to be viewed by the user is increased. Therefore, information with higher importance can be easily provided to the user while improving the visibility of the vital information displaying apparatus 1 on which temporal changes of measured values of a plurality of vital parameters are indicated as a movement of a point on the coordinate plane 20.

Alternatively, in the display controlling section 13, only one of the first and second arrows 61, 62 which corresponds to the larger one of the first and second amounts of changes may be caused to be displayed on the displaying section 12.

In an example illustrated in Fig. 8C, for example, the first amount of variation is larger than the second amount of variation. Therefore, the display controlling section 13 causes only the first arrow 61 to be displayed on the displaying section 12.

With this configuration, only the amount of variation of a vital parameter of higher importance can be presented to the user. Therefore, information with higher importance can be provided to the user while improving the visibility of the vital information displaying apparatus 1 on which temporal changes of measured values of a plurality of vital parameters are indicated as a movement of a point on the coordinate plane 20.

In the embodiment, the functions of the measured value acquiring section 11, the display controlling section 13, and the parameter setting section 14 are implemented by software executed by a cooperation of a processor and memory which are communicably connected to each other. Examples of the processor are a CPU and an MPU. Examples of the memory are a RAM and a ROM. However, at least one of the measured value acquiring section 11, the display controlling section 13, and the parameter setting section 14 may be implemented by hardware such as circuit devices, or a combination of hardware and software.

The above-described embodiments are mere examples for facilitating understanding of the disclosure. The configurations of the embodiments may be adequately changed or improved without departing the concept of the disclosure.

In the above-described embodiments, the circular symbols are used as the first and second plots 31, 32. However, the forms of the first and second plots 31, 32 may be adequately selected as far as the forms can indicate measured values of the first and second vital parameters at respective time points.

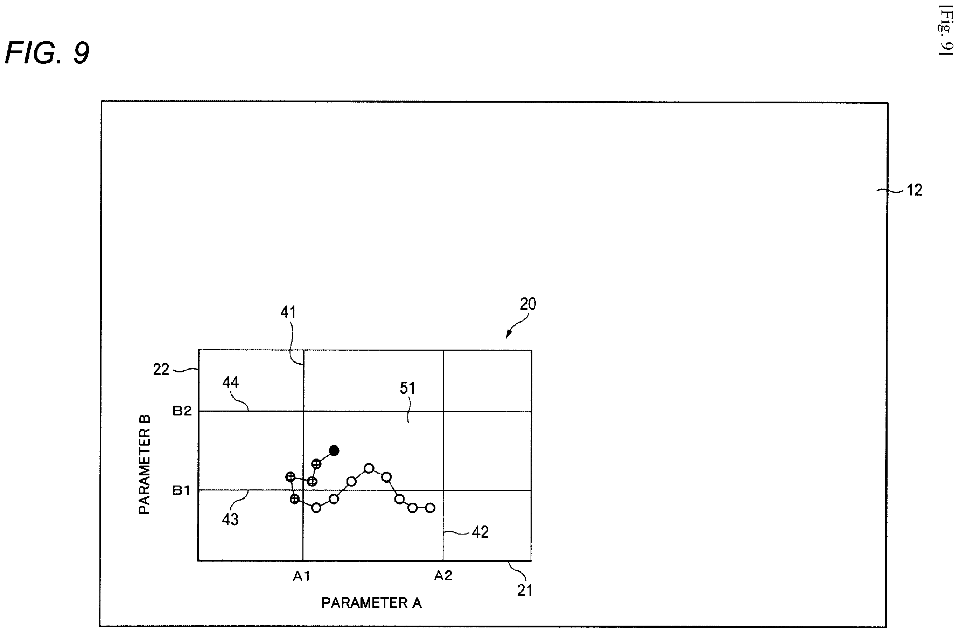

In the above-described embodiments, as illustrated in Fig. 2, the coordinate plane 20 is displayed so as to occupy a substantially whole of the displaying section 12. Alternatively, as illustrated in Fig. 9, the coordinate plane 20 may be displayed so as to occupy an arbitrary part of the displaying section 12. Further alternatively, the coordinate plane 20 may be displayed in a pop-up manner in a state where other vital information is displayed on the whole of the displaying section 12. Also in the alternatives, the region 51 which is defined inside the first, second, third, and fourth reference lines 41, 42, 43, 44 is displayed in a portion including the center of the coordinate plane 20.

In the above-described embodiments, the arrow 60, the first arrow 61, and the second arrow 62 are used as the symbols indicating the first amount of variation of the first vital parameter, and the second amount of variation of the second vital parameter. However, a form of an at least one of the arrow 60, the first arrow 61, and the second arrow 62 may be appropriately selected as far as the form can indicate the absolute value and increase or decrease of the amount of variation of the corresponding parameter.

In the above-described embodiments, the first and second reference values of the first vital parameter correspond respectively to the lower and upper limit of the range in which the value of the first vital parameter is deemed to be normal. However, the definitions of the first and second reference values of the first vital parameter may be adequately selected in accordance with the kind of the first vital parameter, and the condition of the subject.

In the above-described embodiments, the first and second reference values of the second vital parameter correspond respectively to the lower and upper limit of the range in which the value of the second vital parameter is deemed to be normal. However, the definitions of the first and second reference values of the second vital parameter may be adequately selected in accordance with the kind of the second vital parameter, and the condition of the subject.

The present application is based on Japanese Patent Application No. 2015-215742 filed on November 2, 2015

## Claims

1. A vital information displaying apparatus (1) comprising:
a displaying section (12);
a display controlling section (13) configured to cause the displaying section to display a coordinate plane (20) formed by a first coordinate axis extending in a first direction and a second coordinate axis extending in a second direction perpendicular to the first direction; and
a parameter setting section (14) configured to selectively determine a first vital parameter to be associated with the first coordinate axis and a second vital parameter to be associated with the second coordinate axis,
wherein the display controlling section is configured to cause items to be displayed on the coordinate plane in the displaying section, the items including:
a first reference line (41) indicating a first reference value of the first vital parameter and extending in the second direction;
a second reference line (42) indicating a second reference value of the fist vital parameter and extending in the second direction;
a third reference line (43) indicating a third reference value of the second vital parameter and extending in the first direction;
a fourth reference line (44) indicating a fourth reference value of the second vital parameter extending in the first direction;
a first symbol (31) indicating a combination of a value of the first vital parameter and a value of the second vital parameter that are measured at a first time point; and
a plurality of second symbols (32) each indicating a combination of a value of the first vital parameter and a value of the second vital parameter that are measured at one of second time points that are before the first time point;
wherein the display controlling section is configured to cause a region (51) defined inside the first reference line, the second reference line, the third reference line and the fourth reference line to be displayed on such a position that includes a center of the coordinate plane, irrespective of kinds of the first and second vital parameter determined by the parameter setting section, and the first to fourth reference values are variable in accordance with user input;
wherein the display controlling section is configured to cause a third symbol (60, 61, 62) to be displayed on the displaying section, the third symbol indicating a first amount of variation of the value of the first vital parameter during a time period from one of the second time points to the first time point and/or indicating a second amount of variation of the value of the second vital parameter during the time period;
wherein the third symbol is at least one of:
a first arrow indicating the first amount of variation and extending in the first direction;
a second arrow indicating the second amount of variation and extending in the second direction; and
a third arrow connecting the first symbol and one of the second symbols corresponding to the one of the second time points.

2. The vital information displaying apparatus of claim 1,
wherein the display controlling section is configured to differentiate a display mode of the region from a display mode of other portions of the coordinate plane.

3. The vital information displaying apparatus of claim 1,
wherein the display controlling section is configured to differentiate a display mode of at least one of nine regions defined in the coordinate plane by the first reference line, the second reference line, the third reference line and the fourth reference line from other portions of the coordinate plane in accordance with a user input.

4. The vital information displaying apparatus of any one of claims 1 to 3,
wherein the display controlling section is configured to change color of at least one of the second symbols in accordance with a time period until the first time point.

5. The vital information displaying apparatus of claim 1,
wherein the display controlling section is configured to change color of the first arrow in accordance with the first amount of variation as well as color of the second arrow in accordance with the second amount of variation.

6. The vital information displaying apparatus of claim 1,
wherein the display controlling section is configured to cause the third arrow to be displayed with color corresponding to larger one of the first amount of variation and the second amount of variation.

7. The vital information displaying apparatus of claim 1,
wherein the display controlling section is configured to display either one of the first arrow and the second arrow that corresponds to larger one of the first amount of variation and the second amount of variation.

8. The vital information displaying apparatus of claim 1, wherein
the first to fourth lines are displayed inside an outer frame of the coordinate plane.

## Patentansprüche

1. Vorrichtung (1) zum Anzeigen von Vitalinformationen, die umfasst:
einen Anzeigeabschnitt (12);
einen Anzeige-Steuerungsabschnitt (13), der so konfiguriert ist, dass er den Anzeigeabschnitt veranlasst, eine Koordinatenebene (20) anzuzeigen, die durch eine erste Koordinatenachse, die sich in einer ersten Richtung erstreckt, und eine zweite Koordinatenachse gebildet wird, die sich in einer zweiten Richtung senkrecht zu der ersten Richtung erstreckt, sowie
einen Parameter-Einstellabschnitt (14), der so konfiguriert ist, dass er selektiv einen der ersten Koordinatenachse zuzuordnenden ersten Vitalparameter sowie einen der zweiten Koordinatenachse zuzuordnenden zweiten Vitalparameter bestimmt,
wobei der Anzeige-Steuerungsabschnitt so konfiguriert ist, dass er veranlasst, dass Elemente auf der Koordinatenebene in dem Anzeigeabschnitt angezeigt werden, wobei die Elemente einschließen:
eine erste Bezugslinie (41), die einen ersten Bezugswert des ersten Vitalparameters zeigt und sich in der zweiten Richtung erstreckt;
eine zweite Bezugslinie (42), die einen zweiten Bezugswert des ersten Vitalparameters zeigt und sich in der zweiten Richtung erstreckt;
eine dritte Bezugslinie (43), die einen dritten Bezugswert des zweiten Vitalparameters zeigt und sich in der ersten Richtung erstreckt;
eine vierte Bezugslinie (44), die einen vierten Bezugswert des zweiten Vitalparameters zeigt und sich in der ersten Richtung erstreckt;
ein erstes Symbol (31), das eine Kombination aus einem Wert des ersten Vitalparameters und einem Wert des zweiten Vitalparameters zeigt, die zu einem ersten Zeitpunkt gemessen werden; sowie
eine Vielzahl zweiter Symbole (32) die jeweils eine Kombination aus einem Wert des ersten Vitalparameters und einem Wert des zweiten Vitalparameters zeigen, die zu einem von zweiten Zeitpunkten gemessen werden, die vor dem ersten Zeitpunkt liegen;
wobei der Anzeige-Steuerungsabschnitt so konfiguriert ist, dass er veranlasst, dass ein Bereich (51), der innerhalb der ersten Bezugslinie, der zweiten Bezugslinie, der dritten Bezugslinie und der vierten Bezugslinie definiert ist, unabhängig von Typen des ersten und des zweiten Vitalparameters, die durch den Parameter-Einstellabschnitt bestimmt werden, an einer Position angezeigt wird, die einen Mittelpunkt der Koordinatenebene einschließt, und der erste bis vierte Bezugswert Benutzereingabe gemäß variieren können;
wobei der Anzeige-Steuerungsabschnitt so konfiguriert ist, dass er veranlasst, dass ein drittes Symbol (60, 61, 62) auf dem Anzeigeabschnitt angezeigt wird, wobei das dritte Symbol einen ersten Betrag an Variation des Wertes des ersten Vitalparameters während einer Zeitspanne von einem der zweiten Zeitpunkte bis zu dem ersten Zeitpunkt zeigt und/oder einen zweiten Betrag an Variation des Wertes des zweiten Vitalparameters während der Zeitspanne zeigt;
wobei das dritte Symbol ist:
ein erster Pfeil, der den ersten Betrag an Variation zeigt und sich in der ersten Richtung erstreckt;
ein zweiter Pfeil, der den zweiten Betrag an Variation zeigt und sich in der zweiten Richtung erstreckt; oder/und
ein dritter Pfeil, der das erste Symbol und eines der zweiten Symbole verbindet, das dem einen der zweiten Zeitpunkte entspricht.

2. Vorrichtung zum Anzeigen von Vitalinformationen nach Anspruch 1,
wobei der Anzeige-Steuerungsabschnitt so konfiguriert ist, dass er einen Unterschied zwischen einem Anzeigemodus des Bereiches und einem Anzeigemodus anderer Abschnitte der Koordinatenebene macht.

3. Vorrichtung zum Anzeigen von Vitalinformationen nach Anspruch 1,
wobei der Anzeige-Steuerungsabschnitt so konfiguriert ist, dass er einen Unterschied zwischen einem Anzeigemodus wenigstens eines von neun Bereichen, die in der Koordinatenebene durch die erste Bezugslinie, die zweite Bezugslinie, die dritte Bezugslinie sowie die vierte Bezugslinie definiert sind, und anderen Abschnitten der Koordinatenebene entsprechend einer Benutzereingabe macht.

4. Vorrichtung zum Anzeigen von Vitalinformationen nach einem der Ansprüche 1 bis 3, wobei der Anzeige-Steuerungsabschnitt so konfiguriert ist, dass er Farbe wenigstens eines der zweiten Symbole entsprechend einer Zeitspanne bis zu dem ersten Zeitpunkt ändert.

5. Vorrichtung zum Anzeigen von Vitalinformationen nach Anspruch 1,
wobei der Anzeige-Steuerungsabschnitt so konfiguriert ist, dass er Farbe des ersten Pfeils entsprechend dem ersten Betrag an Variation und Farbe des zweiten Pfeils entsprechend dem zweiten Betrag an Variation ändert.

6. Vorrichtung zum Anzeigen von Vitalinformationen nach Anspruch 1,
wobei der Anzeige-Steuerungsabschnitt so konfiguriert ist, dass er veranlasst, dass der dritte Pfeil mit Farbe angezeigt wird, die dem größeren von dem ersten Betrag an Variation und dem zweiten Betrag an Variation entspricht.

7. Vorrichtung zum Anzeigen von Vitalinformationen nach Anspruch 1,
wobei der Anzeige-Steuerungsabschnitt so konfiguriert ist, dass er von dem ersten Pfeil und dem zweiten Pfeil denjenigen anzeigt, der dem größeren von dem ersten Betrag an Variation und dem zweiten Betrag an Variation entspricht.

8. Vorrichtung zum Anzeigen von Vitalinformationen nach Anspruch 1, wobei
die erste bis vierte Linie innerhalb eines äußeren Rahmens der Koordinatenebene angezeigt werden.

## Revendications

1. Appareil d'affichage d'informations vitales (1) comprenant :
une section d'affichage (12) ;
une section de commande d'affichage (13) conçue pour amener la section d'affichage à afficher un plan de coordonnées (20) formé par un premier axe de coordonnées s'étendant dans un premier sens et un second axe de coordonnées s'étendant dans un second sens perpendiculaire au premier sens ; et
une section de réglage de paramètre (14) conçue pour déterminer sélectivement un premier paramètre vital à associer au premier axe de coordonnées et un second paramètre vital à associer au second axe de coordonnées,
la section de commande d'affichage étant conçue pour causer l'affichage d'éléments sur le plan de coordonnées dans la section d'affichage, les éléments incluant :
une première ligne de référence (41) indiquant une première valeur de référence du premier paramètre vital et s'étendant dans le second sens ;
une seconde ligne de référence (42) indiquant une seconde valeur de référence du premier paramètre vital et s'étendant dans le second sens ;
une troisième ligne de référence (43) indiquant une troisième valeur de référence du second paramètre vital et s'étendant dans le premier sens ;
une quatrième ligne de référence (44) indiquant une quatrième valeur de référence du second paramètre vital s'étendant dans le premier sens ;
un premier symbole (31) indiquant une combinaison d'une valeur du premier paramètre vital et d'une valeur du second paramètre vital qui sont mesurées à un premier point dans le temps ; et
une pluralité de seconds symboles (32) indiquant chacun une combinaison d'une valeur du premier paramètre vital et d'une valeur du second paramètre vital qui sont mesurées à l'un des seconds points dans le temps qui se trouvent avant le premier point dans le temps ;
la section de commande d'affichage étant conçue pour amener une région (51) définie à l'intérieur de la première ligne de référence, la seconde ligne de référence, la troisième ligne de référence et la quatrième ligne de référence à s'afficher sur une position telle que celle incluant un centre du plan de coordonnées, quels que soient les types du premier et du second paramètre vital déterminés par la section de réglage de paramètre, et la première à la quatrième valeur de référence étant variables en fonction d'une saisie utilisateur ;
la section de commande d'affichage étant conçue pour causer un affichage d'un troisième symbole (60, 61, 62) sur la section d'affichage, le troisième symbole indiquant une première quantité de variation de la valeur du premier paramètre vital durant une période de temps depuis l'un des seconds points dans le temps jusqu'au premier point dans le temps et/ou indiquant une seconde quantité de variation de la valeur du second paramètre vital durant la période de temps ;
le troisième symbole étant au moins l'un parmi :
une première flèche indiquant la première quantité de variation et s'étendant dans le premier sens ;
une seconde flèche indiquant la seconde quantité de variation et s'étendant dans le second sens ; et
une troisième flèche raccordant le premier symbole et l'un des seconds symboles correspondant à l'un des seconds points dans le temps.

2. Appareil d'affichage d'informations vitales selon la revendication 1,
la section de commande d'affichage étant conçue pour différencier un mode d'affichage de la région d'un mode d'affichage d'autres portions du plan de coordonnées.

3. Appareil d'affichage d'informations vitales selon la revendication 1,
la section de commande d'affichage étant conçue pour différencier un mode d'affichage d'au moins l'une de neuf régions définies dans le plan de coordonnées par la première ligne de référence, la seconde ligne de référence, la troisième ligne de référence et la quatrième ligne de référence des autres portions du plan de coordonnées en fonction d'une saisie utilisateur.

4. Appareil d'affichage d'informations vitales selon l'une quelconque des revendications 1 à 3,
la section de commande d'affichage étant conçue pour changer une couleur d'au moins l'un des seconds symboles en fonction d'une période de temps jusqu'au premier point dans le temps.

5. Appareil d'affichage d'informations vitales selon la revendication 1,
la section de commande d'affichage étant conçue pour changer une couleur de la première flèche selon la première quantité de variation ainsi qu'une couleur de la seconde flèche selon la seconde quantité de variation.

6. Appareil d'affichage d'informations vitales selon la revendication 1,
la section de commande d'affichage étant conçue pour causer l'affichage de la troisième flèche avec une couleur correspondant à une plus grande de la première quantité de variation et de la seconde quantité de variation.

7. Appareil d'affichage d'informations vitales selon la revendication 1,
la section de commande d'affichage étant conçue pour afficher l'une ou l'autre de la première flèche et de la seconde flèche qui correspond à une plus grande de la première quantité de variation et de la seconde quantité de variation.

8. Appareil d'affichage d'informations vitales selon la revendication 1, les première à quatrième lignes étant affichées à l'intérieur d'un cadre externe du plan de coordonnées.
